# EUROPÄISCHE PATENTANMELDUNG

(11) **EP 2 671 615 A1**
(43) Veröffentlichungstag der Anmeldung: **11.12.2013**
(21) Anmeldenummer: 12004267.6
(22) Anmeldetag: 05.06.2012
(51) Int. Cl.: A61Q 19/10, A61K 8/60, A61K 8/73, A61K 8/42, A61K 8/02, A61K 8/96, A61K 8/20, A61K 8/65

(54) **Fester Badezusatz mit retardiertem olfaktorischem und visuellem Freisetzungswirkungsprofil**

(71) Anmelder: Merz Pharma GmbH & Co. KGaA, 60318 Frankfurt (DE)
(72) Erfinder: Kratzer, Elmar, AT 1080 Wien (AT)
(74) Vertreter: Müller, Claudia

(57) **Zusammenfassung**

Die vorliegende Erfindung betrifft einen Badezusatz auf Basis eines wasserlöslichen Trägers, insbesondere eines wasserlöslichen Salzes wie Meersalz, welcher 2 aufeinander aufbauende Wirkphasen mit sichtbarem Einsetzen der zweiten Wirkphase durch Änderung der Farbe und vorzugsweise auch des Duftes umfasst. Im erfindungsgemäßen Sinn stellen die beiden (erste und zweite) Wirkphasen Trägerpartikel dar, welche Wirkstoff bzw. Farbstoff aufweisen bzw. damit beladen sind, die sich bei der Anwendung zu unterschiedlichen Zeiten lösen. Dies er-folgt durch Einsatz eines Retardierungsmittels bei den Partikeln der zweiten Wirkphase. Somit kann anlässlich der Anwendung ein sowohl visuell als auch sensorisch/olfaktorisch wahrnehmbarer Wirkungswechsel wahrgenommen werden.

## Beschreibung

### Gegenstand vorliegender Anmeldung

Die vorliegende Erfindung betrifft einen Badezusatz auf Basis eines wasserlöslichen Trägers, insbesondere eines wasserlöslichen Salzes wie Meersalz, welcher 2 aufeinander aufbauende Wirkphasen mit sichtbarem Einsetzen der zweiten Wirkphase durch Änderung der Farbe und vorzugsweise auch des Duftes umfasst. Im erfindungsgemäßen Sinn stellen die beiden (erste und zweite) Wirkphasen Trägerpartikel dar, welche Wirkstoff bzw. Farbstoff aufweisen bzw. damit beladen sind, die sich bei der Anwendung zu unterschiedlichen Zeiten lösen. Dies er-folgt durch Einsatz eines Retardierungsmittels bei den Partikeln der zweiten Wirkphase. Somit kann anlässlich der Anwendung ein sowohl visuell als auch sensorisch/olfaktorisch wahrnehmbarer Wirkungswechsel wahrgenommen werden.

### Stand der Technik

Badezusätze sind seit langem bekannt und werden sowohl kosmetisch als auch therapeutisch eingesetzt. Insbesondere sollen balneologische Produkte vor allem Wirkungen auf den gesamten Körper ausüben wie Entspannung, Beruhigung, Regenerierung, Erkältungslinderung, anti-rheumatische Effekte z.B. hinsichtlich der Gelenke und vielerlei mehr. Dabei kann die Haut auch gereinigt werden, sie soll aber vor allem nicht ausgetrocknet, vielmehr gepflegt oder auch regeneriert werden. Dazu sind verschiedene Ausführungsformen bekannt wie flüssige Badezusätze in Form von Badeölen, oder wässrige Badezubereitungen (Emulsionen) bzw. auch feste Zusätze wie Badesalze oder Badeperlen.

So beschreibt die DE 42 05 548 A1 flüssige Badezusätze auf Ölgrundlage, welche bestimmte stabilisierende wasserdispergierbare öllösliche polyoxyethylierte Tenside mit einem HLB-Wert von 6 bis 13 zusammen mit Vesikelbildnern wie z.B. Phospholipiden aufweisen. Derartige Badezusätze bilden nach Einbringen in einen Überschuss von Wasser Badeliposomen aus, die aufgrund des verbesserten Wirkstofftransports günstige hautaktive balneologische Wirkungen haben. Gemäß der EP 0930 064 B1 sind feste Badezusätze in Form von Pulvern oder Granulaten bekannt, welche neben einem Brausesatz einen Mineralsalzgehalt, sowie weiterhin Lipidkomponenten wie hydrierte Öle oder Glyceride, und Tenside sowie ebenfalls vesikelbildende Komponenten aufweisen. Dabei wurde das Problem gelöst, die Ausbildung liposomaler Bestandteile trotz des Vorhandenseins von Tensiden und großer Mengen Elektrolyten (Salze) zu ermöglichen.

Die WO 2011/006616 betrifft balneologische Zubereitungen, insbesondere Bade- und Duschzubereitungen, die als fließfähiges oder flüssiges Prämix-Konzentrat neben natürlichen und/oder synthetischen Lipiden, Tensiden, liposomenbildende Phospholipide in Kombination mit Proteinen (MW bis 30000 Da) aufweisen. Letztere beiden Komponenten bilden bei Kontakt mit einem Überschuss an Wasser unmittelbar hautaktive Proteoliposomen (Proteosomen) mit katalytischer Aktivität für einen verbesserten Wirkstofftransport in die Haut. Sie können direkt oder in verdünnten wässrigen oder in festen Anwendungsformen eingesetzt werden. Die WO 2010/022873 A2 beschreibt isometrische Agglomerate, insbesondere Badeperlen aus 80 bis 97 Gew.% eines festen Trägers wie Kollagenhydrolysat, Gelatine oder Gelatinehydrolysat, Cellulose, Kohlenhydrate, Stärke oder Harnstoff mit bis zu 10% Lipiden, und bis zu 11 Gew.% Tensiden sowie liposomenbildenden Substanzen. Hierbei sind die Träger nicht salzartiger Natur, sondern kolloidal und weisen daher auch gute Adsorptionseigenschaften auf, darüber hinaus werden liposomale Strukturen zur Wirkverbesserung eingesetzt.

In den oben beschriebenen Produkten werden demnach die mit Wirkstoffen erzielbaren balneologischen Effekte insbesondere durch besondere Mechanismen des (vor allem liposomalen) Stofftransports erzielt.

Eine andere Wirkung als die physiologische ist in der DE 10 2009 031 273 A1 beschrieben: hierin werden feste Badezusätze offenbart, welche insbesondere akustische Effekte auslösen. Die beschriebenen Badezusätze in fester Form weisen 0,5-10 Gew.% Zucker wie Glucose und/oder Saccharose, Lactose, 0,01-20 Gew.% Farbstoffe, 0,005-10 Gew.% Pflegestoffe, 0,1-5 Gew.% Parfüme und/oder Aromastoffe, 0,1-20 Gew.% Hilfsstoffe und ad 100 Gew.% Salz auf. Dabei soll der akustische Effekt durch den Zucker erreicht werden, der knisternd gleichzeitig mit den übrigen Substanzen in Lösung geht. Eine solcher Badezusatz eignet sich daher insbesondere zur Anwendung bei Kindern.

Von Vorteil wären jedoch auch Produkte, bei welchen nicht nur zu einem bestimmten Zeitpunkt ein akustischer Effekt erzielt wird, sondern z. B. auch eine Wirkungsänderung, Wirkungsergänzung oder Wirkungsverstärkung im Verlaufe der Anwendung erfolgen bzw. insbesondere optisch/sensorisch wahrgenommen werden kann.

### Aufgabe der Erfindung

Aufgabe vorliegender Erfindung ist es daher, einen Badezusatz bereitzustellen, der einfach herzustellen ist und bei dem die oben beschriebenen physiologischen Wirkungen nicht durch aufwendige Transportmechanismen erzielt werden, sondern durch die Inhaltsstoffe selbst, wobei die Wirkungen im Verlaufe der Anwendung zu verschiedenen Zeiten in unterschiedlicher Ausprägung stattfinden sollten.

### Lösung der Aufgabe

Diese Aufgabe wird erfindungsgemäß gelöst durch die Bereitstellung eines festen 2- Phasen -Badezusatzes auf Basis eines wasserlöslichen Trägers, vor allem auf Salzbasis, insbesondere Meersalzbasis, welcher neben einem oder mehreren geeigneten Wirkstoffen, insbesondere ätherischen Ölen/Extrakten, einen oder mehrere, vor allem auf die Wirkstoffe abstimmbare Farbstoffe aufweist. Dabei sind in einer ersten Phase die Trägerpartikel mit einem oder mehreren Wirkstoffen zusammen mit einem oder mehreren Farbstoffen vorhanden. In einer retardierten zweiten Phase sind die Trägerpartikel, insbesondere die Salzpartikel mit einem anderen Farbstoff, vorzugsweise zusammen mit einem oder mehreren Wirkstoffen vorhanden. Der oder die Wirkstoffe, insbesondere ätherische Öle, verfügen neben den oben beschriebenen physiologischen Effekten zumeist auch über einen olfaktorischen Effekt, so dass durch Einsatz bestimmter Stoffe oder Kombinationen hiervon bestimmte geruchliche Wirkungen im Zusammenhang mit der physiologischen Wirkung erzielt werden können. Durch den erfindungsgemäßen zeitverzögerten Einsatz von Farbstoffen in den 2 verschiedenen Phasen erfolgt somit bei Anwendung ein zeitlich versetzter Farbwechsel, insbesondere auch in Kombination mit einer Änderung, Verstärkung und/oder zusätzlichen physiologischen Wirkung durch Wahl geeigneter Wirkstoffe, vor allem ätherischer Öle. Somit werden mit erfindungsgemäßen 2- Phasen-Badezusätzen 3 Wirkebenen bedient, nämlich die physiologische Wirkung (Aromatherapie), die Duftwirkung und die Farbwirkung.

Erfindungsgemäß bedeutet 2- Phasen- Badezusatz somit einen festen Badezusatz, welcher 2 unterschiedlich beladene Trägerpartikel, insbesondere Salzpartikel aufweist, welche gleichzeitig in die Badeflotte eingetragen werden. Dabei lösen sich die Partikel der ersten Phase, umfassend Wirk- und Farbstoffe, sofort nach dem Eintrag auf (Primärwirkung), während die Partikel der zweiten Phase, umfassend andere Farbstoffe und vorzugsweise Wirkstoffe, erst nach einer zeitlichen Verzögerung eingelöst werden (Sekundärwirkung). Diese zeitliche Verzögerung wird dadurch bewirkt, dass die Partikel der zweiten Phase mit einem Retardierungsmittel, vor allem ausgewählt aus Lipiden, Schellack, Zucker, Gelatine, oder Mischungen hiervon, versehen sind, wodurch das Auflösungsvermögen der Partikel zeitverzögert gesteuert wird.

Die Wirkstoffe, insbesondere ätherischen Öle/Extrakte, der einen ersten Phase können von den Wirkstoffen, die in der anderen zweiten Phase vorhanden sein können, insgesamt oder teilweise verschieden sein.

Besonders bevorzugt sind der oder die Wirkstoffe ausgewählt aus Minzöl, Rosmarinöl, Zitronenöl, Thymianöl, Salbeiöl, Orangenöl, Passionsblumen-Extrakt, Johanniskraut Extrakt, Lavendelöl, Melissenöl, Wacholderöl, Wintergrünöl, Eukalyptusöl, Korianderöl, Ingwerextrakt, Beinwell-Extrakt, Arnika-Extrakt, Kiefernnadelöl, Fichtennadelöl, Ylang Ylang öl, Limettenöl, Mandarinenöl, Grapefruitöl oder Mischungen hiervon.

In einer weiteren geeigneten Ausführungsform ist der Wirkstoff in der ersten Phase ausgewählt aus Minzöl, Rosmarinöl, Zitronenöl, Orangenöl, oder Mischungen hiervon und der Wirkstoff in der zweiten Phase aus Melissenöl, Lavendelöl, Lavandinöl, Lemongrasöl, Calendula äth. oder Mischungen hiervon.

Das Retardierungsmittel der zweiten Phase ist vor allem ausgewählt aus natürlichen Wachsen, festen Ölen, darunter vor allem natürlichen festen Ölen, Schellack oder Kombinationen hiervon.

Dabei ist es von Vorteil, wenn das Retardierungsmittel oder eine Mischung hiervon in einer, oder mehr als einer, insbesondere 2 bis 5, vor allem 2 oder 3 aufeinanderfolgenden Anwendungen (z. B. Schichten auf der Oberfläche) auf dem Träger der zweiten Phase, umfassend einen oder mehrere Farbstoffe sowie vor allem weiterhin einen oder mehrere Wirkstoffe, vor allem ätherische Öle/Extrakte, vorhanden ist.

Als Farbstoffe eigen sich vor allem Indigo, Cochenillerot, Brilliantgrün, Hellgelb, Karminrot, Patentblau, Ponceau, Amaranth, Brillantrosa, Echtgelb, Carotinoide, Rote Beete Extrakt, Chlorophyllin, oder auch Gelb (Riboflavin), Chinolingelb oder Mischungen hiervon.

Mit den erfindungsgemäßen Badezusätzen werden somit mehrere Kernforderungen an ein Bad erfüllt. Zunächst können vor allem insgesamt natürliche, vor allem auch hautschonende Inhaltsstoffe eingesetzt werden. Aufgrund der Abstimmbarkeit von Primär- und Sekundärwirkung kann eine klare Indikation (Muskelbeschwerden, Erkältung, etc., Gelenkwirkung, Entspannung, Beruhigung, Rückenbeschwerden, Hautberuhigung, Hautregenerierung, Stress, Erkältungssymptome, Unruhe, Verspannungen) mit vor allem klarer Wahrnehmbarkeit über die Haut und die Atmung erfolgen. Zusätzlich kann die Farbe des Wassers die Wirkung des/ der Wirkstoffe wie ätherische Öle, (z. B. Lavendel, Melisse und beruhigendes Grün, Blaugrün, Violett gegen Stress, Thymian, Salbei und befreiendes Gelb gegen Erkältungserscheinungen, Rosmarin und anregendes Rot zur Durchblutung, gegen Verspannung) zusammen mit deren typischem Duft (Aromatherapie) das Wohlbefinden spürbar beeinflussen.

### Nähere Erläuterung der Erfindung

Die Erfindung betrifft feste 2- Phasen-Badezusätze, umfassend vorzugsweise bis 97 Gew.%, vor allem 90 bis 95 Gew.% eines wasserlöslichen Trägers, insbesondere Salzträgers, 10 bis 3 Gew.% Zusatzstoffe, ausgewählt aus einem oder mehreren Wirkstoffen, einem oder mehreren Farbstoffen, weiterhin z. B. 0,1 bis 3 Gew.% eines oder mehrerer Retardierungsmittel, ausgewählt aus festen Lipiden, flüssigen Lipiden, Zuckern (Kohlenhydraten), Gelatine, Schellack; sowie galenischen Hilfsmitteln, gewählt aus Konditioniermitteln, Tensidemulgatoren, und Kombinationen hiervon, welche dadurch gekennzeichnet sind, dass die Trägerpartikel einer ersten Phase einen oder mehrere Farbstoffe und einen oder mehrere Wirkstoffe aufweisen, und die Trägerpartikel der zweiten Phase einen oder mehrere Farbstoffe aufweisen, welche(r) unterschiedlich zum Farbstoff/den Farbstoffen der ersten Phase ist/sind, und wobei auf den Trägerpartikeln der zweiten Phase ein oder mehrere Retardierungsmittel aufgebracht sind (insbesondere auf/um ihrer Oberfläche, z. B. als Schicht), und wobei das Mengenverhältnis der Trägerpartikel der ersten Phase zu den Trägerartikeln der zweiten Phase ca. 4 :1 bis 1,2:1, insbesondere 3:1 bis 1,5 : 1 oder auch 2:1 bis 1,5:1 beträgt. Besonders bevorzugt ist das Verhältnis von 3:1 bis 1,5:1.

Besonders bevorzugt weisen die Trägerpartikel der zweiten Phase weiterhin einen oder mehrere Wirkstoffe auf.

Als Wirkstoffe für beide Phasen eignen sich insbesondere ätherische Öle/Extrakte aus Pflanzen, hautpflegende Wirkstoffe oder Kombinationen hiervon wie nachfolgend beschrieben. Dabei können der oder die Wirkstoffe der ersten Phase gleich oder verschieden jenem/n der zweiten Phase sein. Bevorzugt liegen Kombinationen, insbesondere unterschiedliche Kombinationen von Wirkstoffen in den beiden Phasen vor. Insbesondere bevorzugt ist mindestens ein Wirkstoff der zweiten Phase nicht gleich mit einem Wirkstoff der ersten Phase.

Als Trägergrundlage werden bevorzugt Salze, ausgewählt aus Mineralsalzen, Solesalzen, Totes Meersalz, Carbonatsalz (Brausesatzsalz), Meersalz, insbesondere natürliches Meersalz oder Mischungen hiervon eingesetzt.

Andere Trägermaterialien sind z. B. ausgewählt aus Zuckern wie Fructose, Saccharose, Dextrose, Stärke, insbesondere Kartoffelstärke oder auch Harnstoff oder Mischungen wie nachfolgend beschrieben.

Als Retardierungsmittel werden besonderes bevorzugt Lipide, vor allem feste Lipide wie Wachse (Bienenwachs, Canaubawachs, Jojobaöl, Shea Butter), sowie flüssige natürliche Lipide (wie natürliche Öle) und insbesondere Mischungen aus festen und flüssigen natürlichen Lipiden eingesetzt.

Die erfindungsgemäßen Badezusätze können als Pulver, Kügelchen oder Granulat, gewünschtenfalls auch gepresst/tablettiert vorliegen. Insbesondere bevorzugt ist Granulat, vor allem in der zweiten Phase, während die erste Phase auch als Pulver oder Granulat vorliegen kann. Dadurch, dass die Trägerpartikel jeweils unterschiedliche Farbstoffe aufweisen, wird je nach Kombination ein buntes Produkt erhalten. Durch das zeitverzögerte Herauslösen der Wirk- und Farbstoffe wird zunächst eine aromatherapeutisch/sensorische Grund(Primär)-wirkung mit einer bestimmten Farbe durch die Träger- (vor allem Salz-)partikel der ersten Phase erzeugt. Nach einer gewissen Zeit erfolgt ein Farbwechsel durch Lösen der retardierten Partikel der zweiten Phase, wobei auch ein Aromawechsel durch weitere/andere Wirkstoffe in dieser Phase stattfinden kann. Somit kann der Anwender sowohl visuell als auch sensorisch/olfaktorisch eine Wirkungsänderung durch zusätzliche oder verstärkende Effekte wahrnehmen.

Insbesondere kann durch die Wirkstoffe, vor allem ätherischen Öle/Extrakte eine gezielte Wirkung/Linderung von körperlichen Beschwerden wie Erkältungssymptome, Gelenkschmerzen, Rückenschmerzen, Verspannungen, Muskelkater, Hautproblemen, z. B. Hautberuhigung bei Reizungen oder auch Hautregenerierung, in der Primärphase erfolgen.

In der Sekundärphase kann dazu ein verstärkender und/oder zusätzlicher Effekt erzielt werden wie z. B. neben den physischen Beschwerden Linderung psychischer Belastungen wie Stress, Schlaflosigkeit, Kraftlosigkeit, Unruhe, was zu einer tiefen Entspannung führt.

Es war überraschend, dass ein derartiger Effekt bzgl. des getrennten Aroma-/Wirkungs-und vor allem Farbwechsels überhaupt erzielt werden konnte und nicht eine undifferenzierbare Vermengung der Effekte erfolgt, da der Stand der Technik unterschiedliche Effekte überhaupt nicht beschrieben und besondere Effekte nur mit zur selben Zeit gelösten Partikeln, und ohne visuellen Wirkstoffeffekt vorgeschlagen hat. Es wird vermutet, dass die gezielte Auswahl und Anwendung an Retardierungsmitteln, insbesondere den lipidischen Substanzen, zu der Lösung des Problems beiträgt.

### Nähere Beschreibung der Inhaltsstoffe

### 1. Wirkstoffe

Diese können vor allem ausgewählt werden aus ätherischen Ölen/Extrakten bzw. Pflanzenextrakten aus:
Rosmarin, Melisse, Zitrone, Lavendel, Kiefernnadel, Zypresse, Mango, Feigen, Zedernholz, Lotus, Kamille, Ylang Ylang, Orangen, Wasserlilie, Passionsblume, Thymian, Minze, Limetten, Grapefruit, Johanniskraut, Mandarine, Wacholder, Baldrian, Zitronenmelisse, Lavendel, Eukalyptus, Lemongras, Fichtennadel, Salbei, Johanniskraut, Lindenblüten, Teebaumöl, Pfefferminze, Rosenholz, Palmarosa, Cranberry, Granatapfel, Ananas, Guave, Echinacea, Efeublätterextrakt, Heidelbeerextrakt, Kaki, Melonen, Magnolien-, Ananas-Extrakt u. ä. oder Mischungen hiervon wie Mischungen aus Minzöl, Rosmarin, Melisse, Arnika-Extrakt, Calendula äth., Beinwell-Extrakt u.a.

Insbesondere bevorzugt sind: Minzöl, Rosmarinöl, Zitronenöl, Orangenöl, Lavendelöl, Melissenöl oder auch Thymianöl, Salbeiöl, Passionsblumenextrakt, Johanniskraut-Extrakt, oder Mischungen hiervon.

Die Wirkstoffe, insbesondere die ätherischen Öle der ersten Phase sind vor allem die richtungsgebenden Komponenten, welche eine bestimmte physiologisch/sensorisch- aromatherapeutische Grund(Primär-)wirkung entfalten wie Beruhigung, Entspannung, Durchblutung, Muskelrelaxierung, Hautregenerierung, Hautberuhigung, Erkältungslinderung, Gelenkbeeinflussung.

Dabei können folgende Grund(Primär-)wirkungen z. B. durch folgende Wirkstoffe oder Kombinationen hiervon erzielt werden:
Minzöl, Rosmarinöl, Zitronenöl oder Kombinationen hiervon für einen durchblutungsfördernden Effekt;
Thymianöl, Salbeiöl, Minzöl oder Kombinationen hiervon für einen befreienden, insbesondere atembefreienden Effekt;
Orangenöl, Passionsblumen, Johanniskraut- Extrakt für einen beruhigenden, "stimmungsfördernden" Effekt.

Besonders bevorzugt werden in der ersten Phase ein oder mehrere Wirkstoffe, ausgewählt aus Minzöl, Rosmarinöl, Zitronenöl, Orangenöl, Thymianöl, Salbeiöl, eingesetzt.

Die Wirkstoffe, insbesondere ätherischen Öle/Extrakte, der zweiten Phase sind vor allem ausgewählt zur Verstärkung der Primär-(Grund)wirkung und/oder Bewirkung eines zusätzlichen Effekts der Primärwirkung der ersten Phase als Sekundärwirkung in der zweiten Phase. Besonders geeignet sind hier Lavendelöl, Melissenöl, Lemongrasöl, Lavandinöl, Speik-Lavendelöl oder Kombinationen hiervon.

Ganz besonders bevorzugt ist Lavendelöl, welches wirkungsverstärkend und/oder ergänzend eingesetzt werden kann z.B. bzgl. der Primärwirkung Entspannung, Muskelrelaxierung, Erkältungslinderung, bei Stress, Verspannung, Unruhe.

Gegebenenfalls können als Wirkstoffe auch geeignete Mengen hautpflegender Substanzen eingesetzt werden. Dazu gehören z.B. Vitamine wie Vitamin C (-Palmitat, Calciumascorbat/Natriumascorbat, Kaliumascorbat); Vitamine der B-Gruppe wie Thiamin (B1), z. B. Thiaminnitrat, Riboflavin, Niacin/Niacinamid (B3), Pantothensäure (B5) wie Calciumpantothenat, Vitamin H (B7), Biotin oder auch Carotinoide wie Beta/- Alpha-Carotin, Beta-Cryptoxanthin, Lutein, Zeaxanthin, Lycopin.

Carotinoide können gleichzeitig auch als Farbstoffe eingesetzt werden. Gegebenenfalls sind auch Vitamin E (Tocopherol, Tocopherylacetat, Tocopherylpalmitat; Tocopherylsuccinat), Retinol, Retinylpalmitat, Retinylacetat, Retinylproprionat, Vitamin A (Retinol und Derivate hiervon) geeignet.

Weitere geeignete auch hautwirksame Wirkstoffe können gewählt werden aus essentiellen ungesättigten Fettsäuren und deren Estern wie Linol- oder Linolensäure, Glyceryl Linoleate, Glyceryl Linolenate, oder aus auch durchblutungsfördernden Mitteln wie Alpha- und Betahydroxysäuren und deren Derivate, z. B. Glykol-, Äpfel-, Zitronen-, Wein-, Milchsäure, Salicylsäure, Isopropylbenzylsalicylate, C12-13 Alkyl Lactate, Nikotinsäurederivate wie Methyl- oder Tocopherylnikotinat, oder auch aus der Gruppe, umfassend antiphlogistische und antibakterielle Substanzen wie Triterpene (z. B. Ursolsäure, Glycyrrhizinsäure oder Glycyrrhetinsäure und deren Derivate, z. B. Stearyl Glycyrrhetinate, Kaliumglycyrrhinat); oder Pantothensäurederivate, z. B. D- Panthenol, Panthenyltriacetat; oder auch aus Polyphenolen, Flavonoiden (z. B. Ferulasäure und deren Ester, Rutin, oder Isoflavonen wie Soja-Isoflavon oder Coenzym Q 10).

Sofern erforderlich oder gewünscht können auch Haut-Pflegestoffe wie Feuchthaltemittel wie Glycerin, Propylenglykol oder Polyethylenglykole, Polypropylenglykol, Butylenglykol, Sorbitol, Allantoin, Dexpanthenol oder Polymere, z.B. Polyquaternium-Typen, Kollagen oder dessen Hydrolysate, Aminosäuren eingesetzt werden. Proteine oder Proteinhydrolysate, z. B mit einer mittleren Molmasse zwischen 4000 und 30000 Da können ebenfalls eingesetzt werden, sind aber nicht zwingend.

Gegebenenfalls können auch dem Fachmann hierfür bekannte Parfümstoffe, insbesondere natürlichen Ursprungs, hinzugesetzt werden, deren Mischung auf die zu erzielende Wirkung abgestimmt ist.

Die Wirkstoffe liegen bevorzugt in einer Gesamtmenge von 0,5 bis 5 Gew.%, insbesondere 0,5 bis 2 Gew.% vor.

Bevorzugte Wirkstoffe sind ausgewählt aus ätherischen Ölen/Extrakten, insbesondere pflanzlichen Ursprungs, wie oben beschrieben, auch in Kombination mit vor allem wasserlöslichen Vitaminen und/oder wasserlöslichen Haut-Pflegestoffen.

Insgesamt ist es möglich, auf synthetische Wirkstoffe bzw. Inhaltsstoffe, vor allem Parfümstoffe, zu verzichten, da die ätherischen Öle/Extrakte selbst über Duftstoffe verfügen. Auch sind keine Konservierungsmittel erforderlich. Da der Träger, insbesondere das Salz ebenfalls natürlichen Ursprungs ist, und die weiteren Inhaltsstoffe (Farbstoffe, Retardierungsmittel, ggf. galenische Hilfsmittel) gleichfalls aus dieser Kategorie gewählt werden können (siehe nachfolgende Beschreibung), liegt insgesamt ein 2-Phasen-Badezusatz auf natürlicher Basis vor.

### 2. Trägersubstanzen

Als Trägersubstanzen kommen insbesondere Salze oder wasserlösliche Stoffe, ausgewählt aus Kollagenhydrolysat (z. B. mit einer mittleren molaren Masse von maximal 50 000 D, erhältlich aus pflanzlichen, tierischen oder marinen Quellen); Cellulose mit einer mittleren Molekülmasse von 50 000 bis 500 000 D, vorzugsweise 50 000 bis 250 000 D; Kohlenhydrate wie vor allem Saccharose, Fructose, Dextrose, Lactose, Sucrose, Glucose; Stärke (wie z. B. Reis-, Weizen-, Mais- und Kartoffelstärke), oder auch Harnstoff (Urea) infrage.

Geeignete erfindungsgemäße 2- Phasen- Badezubereitungen umfassen solche, worin beide Phasen unterschiedliche Trägermaterialien umfassen wie z.B. Salz/Harnstoff oder Salz/Zucker (Kohlenhydrat), sowie solche, worin beide Phasen die gleichen Trägerpartikel umfassen.

Bevorzugt sind Harnstoff, Kollagenhydrolysat, Kohlenhydrate, Cellulose in einer Phase jeweils mit einem anderen (und nicht mit jeweils dem gleichen) Trägerstoff in der anderen Phase kombiniert.

Vor allem bevorzugt sind Salzträger, insbesondere für beide Phasen, wobei in beiden Phasen jeweils die gleichen Salze oder verschiedene Salze oder Salzgemische vorliegen können. Ganz besonders bevorzugt ist Meersalz, auch für beide Phasen.

Es können die Trägerpartikel der ersten Phase auch ausgewählt sein aus einem oder mehreren Salzen und die Träger der zweiten Phase aus Harnstoff, einem oder mehreren Zuckern, Stärke oder Zucker/Stärke-Gemischen hiervon. Alternativ ist es möglich, dass die Trägerpartikel der ersten Phase ausgewählt sind aus Harnstoff, einem oder mehreren Zuckern, Stärke oder Zucker/Stärke-Gemischen hiervon die Träger der zweiten Phase aus einem oder mehreren Salzen.

Als Salze können alle für diesen Zweck bekannten Salze verwendet werden wie Mineralsalze, insbesondere Salze aus den Kationen Natrium, Kalium, Magnesium, Calcium, Eisen, Ammonium oder Mangan und den Anionen Chlorid, Fluorid, Sulfat oder Nitrat als solche oder als Mischungen, wobei vor allem Natriumchlorid als Hauptanteil eingesetzt wird wie z. B. Solesalz, Meersalz. Möglich ist auch Totes Meer-Salz.

Gegebenenfalls können auch andere aus dem Thermalbadmilieu bekannte Salze verwendet werden, welche auch die Kationen Lithium, Aluminium, Strontium, und die Anionen Iodid, Bromid, Thiosulfat, aufweisen. Hierdurch können thermalbadanaloge Bedingungen erzielt werden.

Es ist auch möglich, einen Teil des Mineralsalzes oder das Mineralsalz (nahezu) vollständig zu ersetzen durch Carbonatsalze (Brausesatzsalz), mit welchen bei der Anwendung durch die CO₂-Entwicklung noch zusätzlich ein Sprudeleffekt erzielt werden kann.

Vorzugsweise besteht der Brausesatz hierbei aus Natrium(bi)-, Calcium-, MagnesiumAmmonium- oder Kalium(bi)carbonat oder deren Mischungen und einer oder mehrerer Säuren, die ausgewählt sind aus Zitronensäure, Weinsäure, Fumarsäure, Adipinsäure; Säureanhydride, ausgewählt aus Zitronensäure-, Bernsteinsäureanhydrid oder deren sauren Salzen, ausgewählt aus Natriumdihydrogenphosphat, Natriumfumarat oder deren Mischungen.

Dem Fachmann sind derartige Brausesätze bekannt (siehe EP 0930064).

Ganz besonders bevorzugt, vor allem auch als Träger für beide Phasen, sind Meersalz, Totes Meersalz, Brausesatzsalz, Solesalz oder geeignete Mischungen hiervon. Insbesondere bevorzugt ist Meersalz, vor allem natürliches, oder auch raffiniertes oder teilraffiniertes Meersalz mit einem Hauptanteil an Natriumchlorid wie 85 bis 99%.

Weiterhin bevorzugt ist Meersalz im Gemisch mit 10 bis 80% (bezogen auf den Anteil an Meersalz) an Brausesatzsalz.

Die Menge an Träger, vor allem Salzträger in den erfindungsgemäßen Zubereitungen beträgt insgesamt im allgemeinen 90 bis 97 Gew.%, insbesondere 90 bis 95 Gew.%, vor allem 93 bis 95 Gew.%, bezogen auf die Gesamtzubereitung.

In erfindungsgemäßen 2-Phasen-Badezusätzen werden vor allem folgende Träger verwendet:

| | | | | |
|---|---|---|---|---|
| i) | Phase 1: | Meersalz; | Phase 2: | Meersalz; (jeweils bevorzugt natürlich) |
| ii) | Phase 1: | Meersalz; | Phase 2: | Solesalz; |
| iii) | Phase 1: | Meersalz; | Phase 2: | Dextrose, Sacharose, Fructose oder Gemische hiervon; |
| iv) | Phase 1: | Totes Meersalz; | Phase 2: | Meersalz; |
| v) | Phase 1: | Meersalz; | Phase 2: | Kartoffel-Weizen-Reisstärke oder Gemische hiervon; |
| vi) | Phase 1: | Solesalz | Phase 2: | Harnstoff |
| vii) | Phase 1: | Harnstoff | Phase 2: | Stärke, insbesondere Kartoffelstärke |
| viii) | Phase 1: | Meersalz | Phase 2: | Zucker (z.B. Glucose, Sucrose, oder Gemische hiervon; |
| ix) | Phase 1: | Meersalz | Phase 2: | Harnstoff. |

Die oben genannten Kombinationen können auch umgekehrt eingesetzt werden, so dass die genannten Substanzen der 1. Phase in der 2. Phase und umgekehrt vorliegen können.

Besonders bevorzugte Kombinationen sind die unter i), iv), xi) oder auch viii) genannten.

### 3. Retardierungsmittel

Insbesondere bevorzugt sind lipidische Retardierungsmittel, darunter vor allem feste Wachse und flüssige Lipide. Damit können dann zusätzlich zu den oben beschriebenen Wirkungen noch weitere hautpflegende Effekte erzielt werden.

Die flüssigen Lipide können ausgewählt werden aus natürlichen flüssigen (gesättigten, ungesättigten) Ölen wie Avocadoöl, oder Illipé Butter, Distelöl, Kokosnussöl, Maiskeimöl, Walnussöl, Palmöl, Macadamianussöl Olivenöl, Sonnenblumenöl, Sojaöl, Pfirsichkernöl, Aprikosenkernöl, Traubenkernöl, Ricinusöl, Erdnußöl, Mandelöl, Nerzöl, Weizenkeimöl, Sesamöl, Palmkernöl, Nachtkerzenöl, Haselnussöl, Hagebuttenkernöl, Baumwollsamenöl, Papayaöl oder anderen in diesem Zusammenhang bekannten Ölen, oder Mischungen hiervon.

Besonders bevorzugte flüssige Öle sind Distelöl und Macadamianussöl.

Andere geeignete flüssige Lipide sind z. B: C₈₋₂₂-Fettalkohole wie Oleylalkohol, Octyldodecanol, Cetyl/stearylalkohol. Diese können vor allem zusätzlich zu anderen Retardierungsmitteln vorhanden sein.

Auch C₁₂₋₂₂ - Fettsäurepartialester von mehrwertigen C₂₋₆- Alkoholen wie insbesondere Diglyceride z. B. Glyceroldistearat; Polyol C₁₂₋₂₂ - Fettsäureester wie Propylenglykol -Dicaprylate / dicaprate, Gemische wie Hexyldecanol und Hexyldecyl Laurat können eingesetzt werden. Das sind z.T. auch Emulgatoren.

Eine weitere Gruppe geeigneter Retardierungsmittel umfasst (insbesondere überhitzte) Zucker wie Glucose, Lactose, Saccharose, Gelatine geeigneter Art oder Schellack.

Eine andere Gruppe geeigneter und vor allem bevorzugter Retardierungsmittel sind Lipide, die ausgewählt sind aus festen, wachsartigen Lipiden wie z. B. Jojobaöl (flüssiges Wachs), Bienenwachs, Canaubawachs, festen Ölen wie Kakaobutter, Mangobutter, oder auch Sheabutter.

Besonders bevorzugte Retardierungsmittel sind natürliches Bienenwachs, Canaubawachs, Kakaobutter, Mangobutter, Shea Butter oder Mischungen hiervon, insbesondere Bienenwachs.

Insbesondere bevorzugt werden natürliche Retardierungsmittel, ausgewählt aus festen bis wachsartigen Lipiden, flüssigen natürlichen Ölen oder vor allem auch Kombinationen hiervon, wobei sich diese auf Mischungen der Retardierungsmittel oder eine Folgeapplikation (Nacheinanderauftrag, z.B. auf der Oberfläche der Trägerpartikel) beziehen können.

In einer weiteren bevorzugten Ausführungsform können die bevorzugten festen/wachs-artigen lipidischen Retardierungsmittel auch mit einem oder mehreren (vor allem überhitzten) Zuckern, wie Glucose, Saccharose, Lactose, kombiniert eingesetzt werden, wobei bevorzugt ein Überschuss an lipidischem Retardierungsmittel vorliegt.

Die bisher in festen Badezubereitungen wie oben beschrieben eingesetzten vesikelbildenden Lipide müssen erfindungsgemäß nicht vorhanden sein, wie vor allem Phospholipide, wie Lecithin (z.B. Ei- oder Soja-Lecithin), oder Phosphatidylcholin, -serin oder -diethan-olamin, Phosphatidylserin, Phospatidylinosit, z. B. aus Soja, Raps, Baumwollesamen, Ei, sowie deren Mischungen. Weitere geeignete Komponenten zur Bildung von Vesikeln sind Sphingolipide (z.B. Ceramide, Cerebroside, Sphingosin, Sphingomyelin), oder auch ethoxylierte Sterole wie Phytosterole (Gemische aus Sistosterol, Camposterol und Stigmasterol),z.B. Ethoxylate hiervon wie PEG-5-Sjabean Sterol oder PEG-5 Rapeseed Sterol, =Generol® 122 E5 bzw. Generol® R E5.

Erfindungsgemäße Zubereitungen sind daher bevorzugt frei von derartigen Vesikelbildnern wie Phospholipiden, Sphingolipiden, oder auch ethoxylierten Sterolen/Phytosterolen.

Erfindungsgemäße 2-Phasen-Badezubereitungen können daher vorzugsweise aus dem/den Trägermaterialien, dem/den Wirkstoffen und Farbstoffen, dem oder den Retardierungsmitteln sowie dem/n genannten galenischen Hilfsmittelstoff(en) bestehen.

Das/die Retardierungsmittel werden in einer Menge, bezogen auf die Gesamtzubereitung, von ca. 0,5 bis 3 Gew.%, insbesondere 0,5 bis 1,8 Gew.% eingesetzt. Es/sie liegen bevorzugt in einem Umfang (Art/ Menge) vor, dass sich die Salzpartikel der zweite Phase etwa 3-5 Minuten nach Eintrag in die Badeflotte und somit nach Eintrag der Salzpartikel der ersten Phase lösen (da erste und zweite Phase gleichzeitig eingebracht werden). Das/die Retardierungsmittel sind auf/um die Trägerpartikel (z. B. als hüllende Schicht, welche z.B. bei der Herstellung durch Sprühcoating erstehen kann) aufgebracht.

Die Retardierungsmittel weisen jeweils dem Fachmann bekannte Löslichkeiten in Wasser auf. Somit kann der Fachmann durch Wahl der jeweiliges Retardierungsmittel oder Retardierungsmittelgemische oder schichtenweise Applikation des/der Retardierungsmittel den gewünschten Lösezeitpunkt bzgl. des Wirkungswechsels, insbesondere des Farb- und Wirkstoffwechsels bestimmen, z. B. zwischen 2 und 15 Minuten oder bevorzugt wie oben angegeben.

Besonders bevorzugt werden die einzelnen Substanzen in der zweiten Phase getrennt retardiert (z. B. durch Sprühcoating). Getrennte Retardierung kann durch Auftragen von mehreren Schichten eines oder verschiedener Retardierungsmittel erfolgen. Durch Retardierung in mehreren Schichten können Farb- bzw. Wirkstoffe, insbesondere ätherischen Öle/Extrakte, der beiden Phasen noch besser getrennt voreinander freigesetzt werden, wodurch überraschender Weise eine klare Trennung der einzelnen beschriebenen sensorisch wahrnehmbaren Effekte (Duft, Aromawirkung) untereinander möglich wird, und zwar sowohl hinsichtlich des visuellen Farbwechsels an sich zwischen der ersten und der zweiten Phase als auch bezüglich des Wirkstoffwechsels. Die getrennt retardierten Träger-, vor allem Salzpartikel, umfassen dann Wirkstoff/e, Farbstoff/e, Retardierungsmittel in unterschiedlicher oder gleicher Reihenfolge. Diese Reihenfolge (Wirkstoff, Farbstoff, Retardierungsmittel) richtet sich nach der Beschaffenheit und der damit verbundenen Auflösungsgeschwindigkeit der Substanzen. So kann z.B. zunächst der Wirkstoff (z.B. ätherisches Öl/Extrakt), dann Retardierungsmittel (z.B. Bienenwachs), dann Farbstoff, Retardierungsmittel in aufeinanderfolgenden Schichten (durch Sprühauftragung) vorhanden sein.

Alternativ und besonders bevorzugt können Farb- und Wirkstoff/e gemeinsam auf die Trägerpartikel aufgetragen sein und diese dann in mehreren Anwendungen (wie Schichten), z. B. 2 bis 5, mit Retardierungsmitteln, z. B. mit gleichen oder insbesondere mit verschiedenen Retardierungsmitteln, versehen sein. Aufgrund der unterschiedlichen Beschaffenheit der Substanzen kann dadurch eine getrennte Freisetzung von Farbstoff und Wirkstoff auf sehr effektive Weise erfolgen. Besonders bevorzugt sind auf die mit Farb-/Wirkstoff und ggf. galenischen Hilfsmitteln versehenen Trägerpartikel der zweiten Phase mehrere unterschiedlichen Retardierungsmittel (in Schichten, nacheinander) aufgebracht wie z.B. flüssige Öle (z. B. Kakaobutter), Wachse (Bienenwachs, Canaubawachs), Zucker/Schellack.

### 4. Farbstoffe

Die Farbstoffe werden ausgewählt aus den hierfür dem Fachmann bekannten Substanzen, insbesondere natürlichen Farbstoffen. Sie werden vor allem entsprechend der gewünschten Grundausrichtung bzw. im Zusammenhang mit der beabsichtigten Primärwirkung ausgewählt. So kann für einen beruhigen/lindernden Effekt zunächst die Farbe Gelb (Riboflawin-5-phos-phat) in der ersten Phase und die Farbe Blau (z. B. Indigo, Patentblau) in der zweiten Phase gewählt werden, was einen Retardierungsfarbumschlag nach Grün bewirkt. Zur Anregung/Durchblutung in der ersten Phase kann ein Rotton (Cochenillerot, Karminrot) gewählt werden, der mit einem Blauton (z. B. Patentblau) in der zweiten Phase zu einem Violettton führt. Der Fachmann kann daher geeignete Kombinationen auswählen, je nach beabsichtigtem Primär/Sekundär- bzw. Gesamteffekt. Bevorzugt für den Farbumschlag sind Primär-Farben (Rot, Gelb, Blau), von deren Basis ausgehend sich der Farbwechsel optimal realisieren lässt, je nach gewünschtem Ziel. Der Farbumschlag kann auch mit Misch-Farben oder Abstufungen der Primärfarben realisiert werden.

Zu den geeigneten Farbstoffen gehören z.B.:
Chinolingelb, Chlorophyll, Brilliantgrün, Rote Beete, Cochenillerot, Indigoblau, Gelb (Ribofiavin,-5-Phosphat-Natrium), Holunderextrakt (Anthocyane), Carotinoide, Vitamin E, Patent- Blau, Hellgelb (z.B. Sudan), Echtgelb, Rot 10B, Brillantrosa, Amaranth, Ponceau, Karminrot.

Besonders bevorzugt sind: Patentblau, Chinolingelb und Amaranth.

Die genannten Farbstoffe sind insbesondere natürlichen Ursprungs und ungiftig bzw. auch in der Lebensmittelindustrie zulässig.

### 5. Galenische Hilfsmittel

Zu den galenischen Hilfsmitteln gehören beispielsweise Konditioniermittel wie Mono- und Dialkylphosphate oder Cetylalkohol o. ä. oder Mischungen hiervon, Kolloide, z.B. Polysaccharide (wie Xanthan Gum), Dextrine, Cyclodextrin oder ähnliche kolloide Saccharide, ferner hochdisperse Kieselsäureverbindungen wie Siliciumdioxid (Silica), Stärkeprodukte wie Reis-, Weizen-, Mais- und Kartoffelstärke (Tapioka), ferner auch hydrophobisch modifizierte Stärken, ferner Lösungsvermittler wie Ethanol, Isopropanol, (Poly)Ethylen-glykol, (Poly)propylenglykol, und ähnliche.

Besonders bevorzugt sind hier natürliche Substanzen wie Stärkeprodukte, vor allem Tapioka, Silica oder Mischungen hiervon.

Das oder die Konditioniermittel sind bevorzugt in Mengen von bis zu 1 Gew.% vorhanden. Das oder die Konditioniermittel können in beiden Phasen des Badezusatzes, bevorzugt jedoch in der zweiten Phase vorhanden sein.

Eine weitere Gruppe erfindungsgemäß einsetzbarer galenischer Zusatzstoffe sind Emulgatoren, die in geeigneten Mengen hinzugesetzt werden können. Dazu gehören vor allem nicht ionische Tensidemulgatoren, anionische Tensidemulgatoren oder Mischungen hiervon.

Nichtionische Tenside werden bevorzugt auf Fettsäure- oder Fettalkoholbasis gewählt wie nicht ionische ethoxylierte und/oder propoxylierte Fettalkohole (Fettalkoholether), nichtionische ethoxylierte und/ oder propoxylierte Fettsäuren (Ester) oder deren Amide wie z. B. Mono- oder Diethanolamide eingesetzt werden, wobei der Fettalkohol bzw. die Fettsäure eine Kettenlänge C₈ bis C₂₀, bzw. C₁₂ bis C₂₂; vor allem C₁₂ bis C₁₈ aufweist. Hierzu gehören vor allem C₁-C₈-Alkyl-C₁₀-C₁₈-Fettalkoholethoxylate mit einem Ethoxylierungsgrad (EO), einem Propoxylierungsgrad (PO) oder einem Gemischtoxylierungsgrad (EO/PO) zwischen 1 und 5 (und einem HLB- Wert von weniger als 10, vor allem weniger als 8) wie Polyoxyethylenlaurylether (1 bis 4 EO, Laureth 1-4), Polyoxyethylen (5)-Oleylether, Polyoxyethylen(7)palmitylether, Polyoxyethylen(15) stearylether. Bevorzugt sind ethoxylierte Produkte.

Dazu gehören weiterhin derartige nichtionische Tenside mit höherem Oxylierungsgrad und entsprechend höherem HLB- Wert von mehr als 10 wie von 11-40, vor allem 11-25, insbesondere 13 bis 20. Insbesondere zählen hierzu höherpolyoxyethylierte/und/oder -propoxylierte C₈₋₂₂-Fettalkohole (vor allem Alkyl-C₁₀-C₁₈-Fettalkoholalkoxylate) oder derartige ethoxylierte und/ oder propoxylierte C₁₂₋₂₂-Fettsäuren mit Ethoxylierungs- und/ oder Propoxylierungsgraden von 8-25 oder Mischungen hiervon wie (HLB- Werte in Klammern): Polyoxypropylen-(15)-stearylether oder, Polyoxyethylensorbitanmonoole-at(15,0), Polyoxyethylensorbitanmonostearat - oder -palmitat 15,0), Polyoxyethylen-stearylether (15,3), Polyoxyethylenoleyether (15,3), Polyoxyethylenoxypropylenmonostearat (15,7), Polyoxyethylenfettalkoholether (16,0), Polyoxyethylenmonostearat (HLB 15) u.ä.. Insbesondere bevorzugt sind hier ethoxylierte C₁₂₋₂₂-Fettsäuren oder ethoxylierte C₈₋₂₂-Fettalkohole mit Ethoxylierungsgraden von 10-20 (HLB- Wert z. B. von 13 bis 20, vor allem 13,5 bis 18. Hierzu gehören z.B. die Polyoxyethylenether des Laurylalkohols (Laureth 4, HLB 9,7; Laureth-23 (HLB 16,9), welche unter dem Handelsnamen Brij® bekannt sind, oder auch die Polyoxyethylenether des Cetylalkohols wie Ceteth-2 (HLB 5), Ceteth 10 (HLB 12,9), Ceteth-20 (Cetomacrogol 1000, HLB 15,7), oder auch Cetyl-Stearylether wie Ceteareth-6,20,25 (Cremophor-Produkte, HLB 10-17) u.a..

Ferner geeignet sind Amide der Fettsäuren oder der ethoxylierten Fettsäuren, einer Kettenlänge C₈ bis C₂₀, vor allem C₁₂ bis C₁₈, wie Ethanolamide oder Diethanolamide wie z. B. Kokosfettsäurediethanolamid.

Ebenso geeignet sind nichtionische C₁₂ -C₂₂ Fettsäureether und C₁₂ -C₂₂ Fettsäureester von mehrwertigen Alkoholen wie Glycerol, Sorbitan, wie Mono-, Di- und Triglyceride von C₁₂ -C₂₂ Fettsäuren (Glyceridester) oder C₁₂ -C₂₂ Fettalkoholen (Glyceridether), Mono-, Di- und Tri-/tetra- usw. Sorbitan- C₁₂ -C₂₂ -Fettsäureester oder Sorbitan-C₁₂ -C₂₂ Fettalkoholether, welche ggf. auch oxyliert (ethoxyliert/ propoxyliert, gemischtoxyliert) sein können. Beispiele hierzu ist Glyceryllaureate, Polysorbat (z. B. Tween 20, Tween 60, Tween 21).

Eine weitere Gruppe geeigneter nichtionischer Tenside stellen Zuckerester oder Zuckerether von Glucose, Saccharose, Methylglukose von C₁₂-C₂₂ -gesättigten, ungesättigten, partial gesättigten Fettsäuren; oder von polyoxyethylierten und/oder polyoxypropylierten mittelkettigen und höherkettigen C₁₂ -C₂₂ -Fettsäuren (Ester) dar ; bzw. von C₈-C₂₀ gesättigten, ungesättigten, partial gesättigten oder derartigen polyoxyethylierten und/oder polyoxypropylierten Fettalkoholen (Ether). Bei polyoxyethylierten oder polyoxypropylierten Produkten kann der EO (PEG- Produkte) bzw. PO (PPG- Produkte)- Grad zwischen 8 und 40, vorzugsweise 8-25 liegen. Es können auch gemischt polyoxylierte Produkte eingesetzt werden.

Als Zucker eignen sich insbesondere Glukose, Saccharose, Methylglukose. Als Fettsäuren bzw. Fettalkohole sind vor allem organische aliphatische C₁₂-C₂₂-Carbonsäuren bzw. derartige Alkohole geeignet. Hierzu gehören insbesondere Caprylsäure, Caprinsäure, Laurinsäure, Myristinsäure, Palmitinsäure, Stearinsäure, Ölsäure bzw. derartige Alkohole wie Laurylalkohol, Stearylalkohol und auch Mischungen dieser.

Bekannte oxylierte (ethoxylierte, propoxylierte, gemischtoxylierte) Zuckertenside sind z. B. PEG-20 Methylglucose-Sesquistearat, PEG-120-Methlyglucosedioleat bzw. Methyl-Gluceth-10, Methyl-Gluceth-20, PPG-10-Methylglucoseether, PPG-20-Methylglucoseether.

Alternativ und/oder zusätzlich können auch anionische, vor allem wasserlösliche Fettalkoholtenside eingesetzt werden, insbesondere in Kombination mit nichtionischen Tensiden, wie vorgenannte Fettsäure- bzw. Fettalkoholderivate (ethoxylierte bzw. propoxylierte, vorzugsweise ethoxylierte Derivate) oder nichtionische Fettsäureamide. Zu den geeigneten Aniontensid- Fettalkoholtensiden gehören vor allem C₈ bis C₂₂-, vor allem C₁₀ bis C₁₈- Fettalkoholsulfate bzw. C₈₋₂₂-Alkylsulfate bzw. C₈₋₂₂-Alkylethersulfate oder deren Sulfatsalze wie Aminsalze, insbesondere Monoisopropanolaminsulfatsalze (z. B. MIPA Laurethsulfat (Monoisopropanolamine- (EO1-4)- Laurylsulfat, ggf. ethoxylierte Derivate hiervon mit einem EO Grad von 0 bis 4, vor allem 1-4). Beispiele weiterer Salze sind Natriumlaurylsulfat, Natriumlaurylethersulfat.

Andere geeignete anionische wasserlösliche Verbindungen sind (C₁-C₁₈-Alkyl)-C₁₂ -C₂₂- Fettalkohol- Aminosäureester- Verbindungen, insbesondere Salze hiervon, wobei die Aminosäuren ausgewählt sind aus Glycin, Taurin und ander. Diese können als Salze eingesetzt werden wie z. B. Alkali-Erdalkalisalze (Na-/K-Ca/-, Mg-Chloride, -Sulfate,- Phosphate) wie vor allem wie Oleyl-, Lauryl- Taurate, z.B. Natriummethyloleyltaurat.

Ferner geeignet sind auch anionische C₁₂₋₂₂- Sulfo-Succinate wie z.B. Disodium Lauryl Sulfosuccinat (im Handel erhältlich z. B. als Plantaton® SUS).

Der oder die Emulgatortenside, welche ggf. in beiden Phasen des Badezusatzes vorhanden sein können, werden bevorzugt in geringen Mengen eingesetzt, wie 0,01 bis 1 Gew.%. Vorteilhafterweise sind das oder die Emulgatortenside in der ersten Phase inkorporiert. Als wasserlösliche Tenside werden vorzugsweise anionische Substanzen ausgewählt, wie (C₁-C₁₈- Alkyl)- C₁₂ -C₂₂- Fettalkohol- Aminosäureester- Verbindungen oder auch nicht ionische Tenside wie C₁₂ -C₂₂ Fettsäureether und C₁₂ -C₂₂ Fettsäureester von mehrwertigen Alkoholen wie Glycerol-, und insbesondere Sorbitan (Tween- Produkte).

Bei Wahl eines Brausesatzsalze umfasst dieser üblicherweise Säuren/Säureregulatoren wie beispielsweise Zitronensäure, Weinsäure, Fumarsäure, Adipinsäure; Säureanhydride, ausgewählt aus Zitronensäure-, Bernsteinsäureanhydrid oder deren saure Salze, ausgewählt aus Natriumdihydrogenphosphat, Natriumfumarat oder deren Mischungen. Diese können gegebenenfalls auch als galenische Hilfsmittel gelten bzw. eingesetzt werden.

### Bevorzugte Ausführungsformen

Bevorzugte Badezusätze umfassen: (oder vorzugsweise bestehen aus) 90 bis 97 Gew.% eines festen wasserlöslichen Trägers, ausgewählt aus Salzen, Harnstoff, Kollagenhydrolysat, Cellulose, Kohlenhydraten wie Saccharose, Fructose, Dextrose, Lactose, 3 bis 10 Gew.% Zusätze, ausgewählt aus, einem oder mehreren Wirkstoffen, einem oder mehreren Farbstoffen, einem oder mehreren Retardierungsmitteln, ausgewählt aus festen Lipiden, flüssigen Lipiden, überhitzten Zuckern, Gelatine, Schellack, sowie galenischen Hilfsmitteln, ausgewählt aus Konditioniermitteln, Emulgatortensiden, und Kombinationen hiervon, wobei
i) die Trägerpartikel einer ersten Phase ein oder mehrere Farbstoffe und einen oder mehrere Wirkstoffe, vor allem ein oder mehrere ätherische Öle/Extrakte aufweisen, und
ii) die Trägerpartikel der zweiten Phase mindestens einen oder mehrere Wirkstoffe, vor allem ein oder mehrere ätherische Öle/Extrakte, und einen oder mehrere Farbstoffe aufweisen, der/die unterschiedlich ist/ sind zum Farbstoff/ den Farbstoffen der ersten Phase sowie weiterhin ein oder mehrere Retardierungsmittel umfassen, und
iii)das Mengen(Gew.%)-verhältnis der Trägerpartikel der ersten Phase zu den Trägerpartikeln der zweiten Phase ca. 4 :1 bis 1,2:1, insbesondere 1,5 : 1 bis 1,2: 1 beträgt.

Weiterhin bevorzugt sind derartige Zusätze, worin die Trägerpartikel der ersten Phase und die Trägerpartikel der zweiten Phase ausgewählt sind aus einem oder mehreren Salzen; oder worin die Trägerpartikel der ersten Phase ausgewählt sind aus einem oder mehreren Salzen wie erläutert und die Träger der zweiten Phase ausgewählt sind aus Harnstoff, einem oder mehreren Zuckern, Stärke oder Zucker/ Stärke-Gemischen hiervon; oder alternativ worin die Trägerpartikel der ersten Phase ausgewählt sind aus Harnstoff, einem oder mehreren Zuckern, Stärke oder Zucker/Stärke-Gemischen hiervon und die Träger der zweiten Phase ausgewählt sind aus einem oder mehreren Salzen.

In einer weiteren Ausführungsform umfassen die Badezusätze der oben beschriebenen Art in der zweiten Phase mindestens einen Wirkstoff, vor allem mehrere, oder auch alle Wirkstoffe, der/die verschieden ist/sind von den Wirkstoffen der ersten Phase.

Bevorzugt ist das oder die Retardierungsmittel in derartigen Badezusätzen ausgewählt aus natürlichen Wachsen, festen Ölen, natürlichen festen Ölen, Schellack, oder Mischungen hiervon, wobei diese(s) in einer, oder mehr als einer, insbesondere 2 bis 5 Anwendungen (z.B. Schichten), vor allem aufeinanderfolgend ein oder mehrere natürliche Wachse, ein oder mehrere flüssige Öle sowie Schellack (als letzten Auftrag/Schicht) aufgebracht sein kann.

### Herstellung

Die erfindungsgemäßen Zubereitungen werden hergestellt, indem man in einem für derartige Zwecke geeigneten Mischer
1) einen ersten Teil, vor allem mehr als 50 bis z.B. 80 Gew.% der Gesamtmenge an im Wesentlichen wasserfreiem (wasserlöslichen) Träger wie Salz, vor allem natürliches Meersalz, mit dem oder den Wirkstoffen, insbesondere ätherischen Ölen/Extrakten sowie einem oder mehreren Farbstoffen entsprechend der ersten Phase, ggf. zusammen mit einem oder mehreren Konditioniermitteln und/oder Emulgatoren geeigneter Form in an sich bekannter Weise mischt;
2) die Restmenge an Träger, vor allem weniger als 50 Gew.%, mit dem oder den Farbstoffen , und vorzugsweise, und vor allem gleichzeitig, mit einem oder mehreren Wirkstoffen gemäß der zweiten Wirkphase, ggf. mit Konditioniermitteln geeigneter Form in an sich bekannter Weise mischt;
3) die aus Schritt 2 erhaltenen Träger(vor allem Salz)-partikel mit einem oder mehreren Retardierungsmitteln oder einem Retardierungsmittelgemisch, ausgewählt aus festen, insbesondere natürlichen Ölen, insbesondere natürlichen Wachsen, flüssigen natürlichen Ölen, (überhitztem) Zucker, Schellack oder auch Gelatine oder Mischungen hiervon vor allem durch Aufsprühen versieht;
4) die aus Schritt 1) und Schritt 3) erhaltenen Partikel in einem geeigneten Verhältnis zwischen 4:1 bis 1,2 :1, zusammenmischt und
5) den gemäß Schritt 4) erhaltenen Badezusatz in geeignete Behälter wie z. B. Einmal-Sachets oder größere Gebinde abfüllt.

Sofern es erforderlich sein sollte, kann nach dem einen oder anderen Schritt 1) bis 3), vorzugsweise 3) eine Trocknung erfolgen. Sofern eine bestimmte Korngröße des Produktes gewünscht ist, können die jeweiligen Trägerpartikel einer jeweiligen Phase vor der Verarbeitung/Mischung mit den Zusatzstoffen, Hilfsmitteln, in eine gewünschte Größe gemahlen werden. Bevorzugt werden die Trägerpartikel der Phase 2, falls es gewünscht ist, zunächst gemahlen und sodann wie in Schritt 2), 3) beschrieben verarbeitet. Die Partikel der Phase 2 liegen vor allem dann als Granulat vor, die Partikel der Phase 1 können als Granulat, Pulver, Kügelchen vorliegen.

Das fertige Produkt kann sodann in geeignete Behältnisse verpackt werden.

Die Herstellung des Produktes erfolgt im allgemeinen bei Raumtemperatur.

### Anwendung und Anwendungsformen

Die erfindungsgemäßen Zubereitungen können als Pulver, bevorzugt als Granulat in Gebinden mit größeren Mengen zum Mehrfachgebrauch bereitgestellt werden. Bevorzugt sind Einzelgebinde (z. B. enthaltend 50 bis 100 g Badesalzzusatz), z. B. Sachets verschiedener Formen wie länglich, rechteckig, oval, aus flexiblen Materialien wie insbesondere transparenter Folie, geschlossen oder auch perforiert, z. B. in Form von flexiblen Kissen, Schlauchbehältnissen u.ä., die aus geeigneten Materialien wie Baumwolle, Gaze, oder aus anderem für derartige Zwecke geeignetem und bekanntem textilem Kunststoffgewebe bestehen. Die Anwendung von Sachets aus transparenter oder teilweise transparenter Folie führt sogleich zur optischen Wahrnehmung der unterschiedlich gefärbten einzelnen Trägerpartikel wie z.B. Salzkristalle. Denkbar sind auch Packungen, z. B. aus Karton, welche mehrere Einzelsachets enthalten.

Aus dem jeweiligen Gebinde wird die geeignete Menge, welche in Einzelgebinden vorgegeben ist (z. B. 50 bis 100 g), in die Badeflotte auf einmal eingetragen. Es entfaltet sich neben dem durch das/die Wirkstoffe, vor allem ätherischen Öle/Extrakte der ersten Phase jeweils bedingte Aroma ein erster Aroma- und Farbeindruck. Nach Beginn des Bades, vorzugsweise etwa 3-5 Minuten danach, erfolgt der erfindungsgemäße Farbwechsel, insbesondere auch eine Aromaverstärkung/und/ oder ein Aromawechsel aufgrund des/der Wirkstoffe, ätherischen Öle aus der zweiten Phase. Durch eine gezielte Wirkstoff- und Farbkombination kann somit eine Linderung und Entspannung bei körperlichen Beschwerden und psychischen Belastungen erfolgen wie z.B. bei Rückenbeschwerden, Stress, Erkältungssymptomen, Unruhe, Verspannungen, Muskelbeschwerden.

Der Badezusatz ist leicht löslich, so dass nach dem Bade keine wesentlichen Anhaftungen bestehen bleiben und die Wanne leicht ausgewischt/gespült werden kann.

Besonders bevorzugte Anwendungsformen sind teiltransparente längliche Sachets mit Badezusätzen zur Herbeiführung eines muskelentspannenden Effekts mit Minzöl, eines beruhigenden Effekts mit Orangenöl, eines erkältungslindernden Effekts mit Minzöl, Salbei, Thymian in der ersten Phase und Melissenöl, Lavendelöl in der zweiten Phase, eines durchblutungsfördernden, gelenkbeeinflussenden Effekts mit Rosmarin in der ersten Phase und Lavendelöl in der zweiten Phase. Andere Darreichungsformen sind ebenfalls möglich und erfindungsgemäß umfasst.

### Beispiele

Die Erfindung wird anhand der nachfolgenden Beispiele näher erläutert.

### Beispiel 1: Muskel-Entspannungsbad

| Inhaltsstoff Phase 1/2 | Gew.% |
|---|---|
| Phase 1 | |
| Meersalz (natürlich) | 74,00% |
| Rosmarinöl | 0,10% |
| Zitronenöl | 0,06% |
| Karminrot-Farbstoff | 0,03% |
| Tween 20 | 0,30% |

| Phase 2 | |
|---|---|
| Meersalz (natürlich) | 22,50% |
| Lavendelöl | 1,00% |
| Bienenwachs | 0,10% |
| Patentblau | 0,01% |
| Shea Butter | 1,50% |
| Stärke | 0,40% |
| | 100,00% |

### Beispiel 2: Haut-Pflegebad

| Inhaltsstoff Phase 1/2 | Gew.% |
|---|---|
| Phase 1 | |
| Totes Meersalz | 20,00% |
| Meersalz (nat.) | 55,00% |
| Hammamelis-Extrakt | 0,50% |
| Kamillenextrakt | 0,40% |
| Chinolingelb | 0,06% |
| Glyceryl laurate | 0,25% |

| Phase 2 | |
|---|---|
| Harnstoff | 22,50% |
| Jojobaöl | 0,10% |
| Calendulaöl äth. | 0,10% |
| Shea Butter | 1,00% |
| Brillantblau | 0,01% |
| Carnubawachs | 0,08% |
| | 100,00% |

### Beispiel 3: Gelenk- Bad

| Inhaltsstoff Phase ½ | Gew.% |
|---|---|
| Phase 1 | |
| Solesalz | 78,00% |
| Beinwell-Extrakt | 0,10% |
| Rosmarinöl | 0,30% |
| Brillantrosa | 0,02% |
| Tween 20 | 0,30% |

| Phase 2 | |
|---|---|
| Solesalz | 19,00% |
| Avocadoöl/Shea Butter | 1,70% |
| Carnaubawachs | 0,27% |
| Lavendinöl | 0,30% |
| Brillantblau | 0,01% |
| | 100,00% |

### Beispiel 4: Bronchialbad

| Inhaltsstoff Phase 1/2 | Gew.% |
|---|---|
| Phase 1 | |
| Meersalz | 76,00% |
| Salbeiöl | 0,36% |
| Thymianöl | 0,28% |
| Hellgelb | 0,06% |
| Ceteth 10 | 0,40% |
| Phase 2 | |
| Sucrose, Maisstärke, Glucose Kügelchen | 20,60% |
| Melissenöl | 0,05% |
| Lemongrasöl | 0,80% |
| Kakaobutter | 0,20% |
| Überhitzter Zucker | 0,60% |
| Patentblau | 0,05% |
| Silica | 0,30% |
| Stärke | 0,40% |
| | 100,00% |

### Beispiel 5: Hautregenerationsbad

| Inhaltsstoff Phase 1/2 | Gew.% |
|---|---|
| Phase 1 | |
| Harnstoff Perlen | 77,60% |
| Lindenblütenextrakt | 1,00% |
| Parfüm | 0,20% |
| Hellgelb | 0,08% |
| Tween 20 | 0,30% |

| Phase 2 | |
|---|---|
| Sucrose, Maisstärke, Gucose Kügelchen | 19,00% |
| Mandelöl | 0,20% |
| Mandelprotein | 0,20% |
| Shea Butter | 1,00% |
| Parfüm | 0,20% |
| Bienenwachs | 0,20% |
| Ponceau | 0,02% |
| | 100,00% |

## Patentansprüche

1. Fester 2- Phasen-Badezusatz, umfassend:
90 bis 97 Gew.% eines festen wasserlöslichen Trägers, ausgewählt aus Salzen, Harnstoff, Kollagenhydrolysat, Cellulose, Kohlenhydraten wie Saccharose, Fructose, Dextrose, Lactose,
3 bis 10 Gew.% Zusätze, ausgewählt aus:
- einem oder mehreren Wirkstoffen,
- einem oder mehreren Farbstoffen,
- einem oder mehreren Retardierungsmitteln, ausgewählt aus festen Lipiden, flüssigen Lipiden, überhitzten Zuckern, Gelatine, Schellack, sowie
- galenischen Hilfsmitteln, ausgewählt aus Konditioniermitteln, Emulgatortensiden, und Kombinationen hiervon, **dadurch gekennzeichnet, dass**
i) die Trägerpartikel einer ersten Phase einen oder mehrere Farbstoffe und einen oder mehrere Wirkstoffe aufweisen, und
ii) die Trägerpartikel der zweiten Phase mindestens einen oder mehrere Farbstoffe aufweisen, der/die unterschiedlich ist/sind zum Farbstoff/den Farbstoffen der ersten Phase sowie weiterhin ein oder mehrere Retardierungsmittel umfassen, und
iii) das Mengen(Gew.%)-verhältnis der Trägerpartikel der ersten Phase zu den Trägerpartikeln der zweiten Phase ca. 4 :1 bis 1,2: 1, insbesondere 1,5 : 1 bis 1,2:1 beträgt.

2. Badezusatz nach Anspruch 1, **dadurch gekennzeichnet, dass** die Trägerpartikel ausgewählt sind aus Salz oder einem Salzgemisch, ausgewählt aus Meersalz, Totes Meersalz, Solesalz, Carbonat-Brausesatzsalz oder Mischungen hiervon.

3. Badezusatz nach Anspruch 1, **dadurch gekennzeichnet, dass** die Trägerpartikel ausgewählt sind aus Harnstoff, Saccharose, Sucrose, Glucose oder Dextrose oder Gemischen hiervon.

4. Badezusatz nach einem der Ansprüche 1 bis 3, **dadurch gekennzeichnet, dass** die Trägerpartikel der ersten Phase verschieden von oder vorzugsweise gleich den Trägerpartikeln der zweiten Phase sind.

5. Badezusatz nach einem der Ansprüche 1 bis 4, **dadurch gekennzeichnet, dass** die Trägerpartikel der ersten Phase und die Trägerpartikel der zweiten Phase ausgewählt sind aus einem oder mehreren Salzen.

6. Badezusatz nach einem der Ansprüche 1 bis 4, **dadurch gekennzeichnet, dass** die Trägerpartikel der ersten Phase ausgewählt sind aus einem oder mehreren Salzen und die Träger der zweiten Phase ausgewählt sind aus Harnstoff, einem oder mehreren Zuckern, Stärke oder Zucker/Stärke-Gemischen oder Mischungen hiervon.

7. Badezusatz nach einem der Ansprüche 1 bis 4, **dadurch gekennzeichnet, dass** die Trägerpartikel der ersten Phase ausgewählt sind aus Harnstoff, einem oder mehreren Zuckern, Stärke oder Zucker/Stärke-Gemischen oder Mischungen hiervon und die Träger der zweiten Phase ausgewählt sind aus einem oder mehreren Salzen.

8. Badezusatz nach einem der Ansprüche 4 bis 6, **dadurch gekennzeichnet, dass** das Salz ausgewählt ist aus der Gruppe, umfassend Meersalz, Totes Meersalz, Solesalz, Carbonat-Brausesatzsalz oder Mischungen hiervon.

9. Badezusatz nach einem der Ansprüche 1 bis 8, **dadurch gekennzeichnet, dass** er als Wirkstoff ein oder mehrere ätherische Öle/Extrakte aufweist.

10. Badezusatz nach einem der Ansprüche 1 bis 9, **dadurch gekennzeichnet, dass** die zweite Phase weiterhin einen oder mehrere Wirkstoffe, insbesondere ausgewählt aus ätherischen Ölen/Extrakten, aufweist.

11. Badezusatz nach Anspruch 10, **dadurch gekennzeichnet, dass** die zweite Phase mindestens einen Wirkstoff aufweist, der verschieden ist von den Wirkstoffen der ersten Phase.

12. Badezusatz nach einem der Ansprüche 1 bis 11, **dadurch gekennzeichnet, dass** der Wirkstoff in der ersten Phase ausgewählt ist aus Minzöl, Rosmarinöl, Zitronenöl, Orangenöl, oder Mischungen hiervon und der Wirkstoff in der zweiten Phase ausgewählt ist aus Melissenöl, Lavendelöl, Lavandinöl, Lemongrasöl, Calendulaöl äth. oder Mischungen hiervon.

13. Badezusatz nach einem der Ansprüche 1 bis 12, **dadurch gekennzeichnet, dass** das Retardierungsmittel ausgewählt ist aus natürlichen Wachsen, festen Ölen, natürlichen festen Ölen, Schellack, oder Mischungen hiervon.

14. Badezusatz nach einem der Ansprüche 1 bis 13, **dadurch gekennzeichnet, dass** die Träger- insbesondere die Salzpartikel der zweiten Phase, umfassend mindestens einen Farbstoff, und mindestens einen Wirkstoff, das oder die Retardierungsmittel in einer, oder mehr als einer, insbesondere 2 bis 5 Anwendungen (z.B. Schichten) aufweisen.

15. Badezusatz nach Anspruch 14, **dadurch gekennzeichnet, dass** die Träger- insbesondere die Salzpartikel der zweiten Phase, umfassend mindestens einen Farbstoff, und mindestens einen Wirkstoff, als Retardierungsmittel aufeinanderfolgend ein oder mehrere natürliche Wachse, ein oder mehrere flüssige Öle sowie Schellack (als letzten Auftrag/Schicht) aufweisen.

16. Badezusatz gemäß einem der Ansprüche 1 bis 15, **dadurch gekennzeichnet, dass** er im wesentlichen frei ist von vesikelbildenden Lipiden.

17. Badezusatz gemäß einem der Ansprüche 1 bis 16 zum aromatherapeutischen balneologischen Einsatz beim Menschen zur Erzielung eines beruhigenden, entspannenden, erkältungslindernden, und/oder eines Gelenke beeinflussenden Effekts, zur Erzielung einer Hautberuhigung, Hautregenerierung, oder bei Rückenbeschwerden, Stress, Erkältungssymptomen, Unruhe, Verspannungen, Muskelbeschwerden.

18. Verfahren zur Herstellung eines Badezusatzes gemäß einem der Ansprüche 1 bis 17, **dadurch gekennzeichnet, dass**
1) ein erster Teil, vor allem mehr als 50 bis z.B. 80 Gew.% der Gesamtmenge eines im Wesentlichen wasserfreien wasserlöslichen Trägers, vor allem Salzträgers, mit dem oder den Wirkstoffen, insbesondere ätherischen Ölen/Extrakten sowie einem oder mehreren Farbstoffen entsprechend der ersten Phase, ggf. zusammen mit einem oder mehreren Konditioniermitteln und/oder Emulgatortensiden geeigneter Form in an sich bekannter Weise gemischt wird;
2) die Restmenge, vor allem weniger als 50 Gew.%, an Träger, insbesondere Salz, mit dem oder den Farbstoffen, und vorzugsweise einem oder mehreren Wirkstoffen gemäß der zweiten Wirkphase ggf. zusammen mit einem oder mehreren Konditioniermitteln geeigneter Form in an sich bekannter Weise gemischt wird;
3) die aus Schritt 2 erhaltenen Trägerpartikel mit einer oder mehreren Schichten eines Retardierungsmittels oder eines Retardierungsmittelgemischs, ausgewählt aus festen, insbesondere natürlichen Ölen, insbesondere natürlichen Wachsen, flüssigen natürlichen Ölen, Schellack, Zucker oder Mischungen hiervon durch Aufsprühen versehen werden;
4) die aus Schritt 1) und Schritt 3) erhaltenen Partikel in einem geeigneten Verhältnis zwischen 4: 1 bis 1,2 :1, zusammengemischt werden und
5) der gemäß Schritt 4) erhaltenen Badezusatz in geeignete Behälter wie Einmal-Sachets oder größere Gebinde abgefüllt wird.

19. Verwendung eines Badezusatzes gemäß einem der Ansprüche 1 bis 16 zur Erzielung eines aromatherapeutischen balneologischen Effektes wie Beruhigung, Entspannung, Erkältungslinderung, Gelenkbeeinflussung, zur Erzielung einer Hautberuhigung, Hautregenerierung oder bei Rückenbeschwerden, Stress, Erkältungssymptomen, Unruhe, Verspannungen, Muskelbeschwerden bei Menschen.
